(19) Europäisches Patentamt
European Patent Office
Office européen des brevets

(11) Publication number: **0 333 522 B1**

# (12) EUROPEAN PATENT SPECIFICATION

(45) Date of publication of patent specification: **17.08.94**

(51) Int. Cl.⁵: **C07C 235/34**, A61K 31/135, C07C 235/36, C07D 295/18, C07D 207/16, C07D 211/60, A61K 31/16, A61K 31/19, A61K 31/215, A61K 31/40, A61K 31/445

(21) Application number: **89302742.5**

(22) Date of filing: **20.03.89**

## (54) Catechol derivatives and pharmaceutical preparations containing same.

(30) Priority: **18.03.88 JP 63515/88**
**18.03.88 JP 63516/88**
**15.08.88 JP 201865/88**
**15.08.88 JP 201866/88**

(43) Date of publication of application:
**20.09.89 Bulletin 89/38**

(45) Publication of the grant of the patent:
**17.08.94 Bulletin 94/33**

(84) Designated Contracting States:
**CH DE FR GB IT LI NL SE**

(56) References cited:
**EP-A- 0 089 710**
**EP-A- 0 261 977**
**US-A- 4 181 738**
**US-A- 4 529 604**

(73) Proprietor: **MITSUI TOATSU CHEMICALS, Inc.**
**2-5 Kasumigaseki 3-chome**
**Chiyoda-Ku Tokyo 100 (JP)**

(72) Inventor: **Fukazawa, Nobuyuki**
**1541-1-4-4, Yabecho**
**Totsuka-ku**
**Yokohama-shi Kanagawa-ken (JP)**
Inventor: **Otsuka, Kengo**
**314 Yamasaki**
**Kamakura-shi Kanagawa-ken (JP)**
Inventor: **Shimada, Shizuo**
**2141, Tougou**
**Mobara-shi Chiba-ken (JP)**
Inventor: **Miyama, Yukio**
**589-1 Hamasyuku**
**Shirakomachi**
**Chousei-gun Chiba-ken (JP)**
Inventor: **Ikeda, Fumiaki**
**226-1 Takashi**
**Mobara-shi Chiba-ken (JP)**

**EP 0 333 522 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**FEBS LETTERS, vol. 208, no. 2, November 1986, pages 258-262, Federation of European Biochemical Societies; Y. FURUKAWA et al.: "Aliphatic side chain of catecholamine potentiates the stimulatory effect of the catechol part on the synthesis of nerve growth factor"**

Inventor: **Kaiho, Tatsuo**
**3-9-3, Inage**
**Chiba-shi Chiba-ken (JP)**

(74) Representative: **Holdcroft, James Gerald, Dr. et al**
**Graham Watt & Co.,**
**Riverhead**
**Sevenoaks, Kent TN13 2BN (GB)**

## Description

Background of the Invention

1. Field of the Invention

The present invention relates to catechol derivatives and their use as medicines. More particularly, it relates to catechol derivatives having the ability to induce production and secretion of nerve growth factor (hereinafter abbreviated as NGF) in the local tissue of the brain. The invention also relates to prophylactic and therapeutic preparations containing these derivatives as active ingredients for regressive disorders of the central nervous system.

2. Description of the Prior Art

Basic and clinical researches have been intensively promoted in order to establish early diagnosis and etiologic therapy for various senile diseases with the increasing average span of life in the world. Regressive disorders of the central nervous system are also one of the principal research subjects. Senile Dementia of the Alzheimer Type (hereinafter abbreviated as SDAT), also known as Alzheimer Disease, a typical disease in particular, is becoming a serious social problem as a result of its increase primarily in advanced countries as well as the progressive and tragic course of the disease.

Particularly in recent years, many researchers and clinicians have investigated extensively and yet neither fundamental elucidation of the disease nor effective early diagnosis and therapy have been established. Many pathological findings, however, have been accumulated on the direct cause of failure of immediate memory and disorientation which are characteristic early symptoms of SDAT. According to these findings, the cause is a progressive degeneration in magnocellular cholinergic tracts projecting from the basal forebrain into the cerebral cortex and hippocampus which are the centers of memory and learning, and an accompaning dysfunction in this responsible region. In addition, precursors in acetylcholine biosymthesis or inhibitors of choline esterase were actually administered to SDAT patients as an activation treatment for the brain cholinergic neuron. Cases of partial improvement have been reported whereas generally the results have been not as effective as expected.

NGF has been the subject of many studies since its idscovery by R. Levi-Montalcini and S. Cohen et al. It has already been demonstrated by several experiments in physiological chemistry that NGF is an essential factor for the peripheral nervous system relating to differentiation and growth of sensory and sympathetic nerves in the fetus and further, to the survival and maintenance of functions in the sympathetic neurons of an adult.

NGF, however, is a potent biologically active substance even in ultra trace amounts. In spite of long term studies, precise information has not been obtained on its distribution in the tissue and movement which directly prove vital functions. Most recently, development and improvements have been made using the highly sensitive enzyme linked immunosorbent assay (hereinafter abbreviated as ELISA) to identify the active subunit of NGF, e.g. $\beta$-NGF (hereinafter simply referred to as NGF). Thus satisfactory detection-sensitivity and specificity for this examination have been attained [S. Furukawa et al., J. Neurochem., 40, 734-744 (1983); S. Korshing and H. Thoenen, Proc. Natl. Acad. Sci. USA, 80, 3513-3516 (1983)].

Further, the NGF gene has been cloned and structure is analyzed. A method for determining messenger RNA (hereinafter abbreviated as m RNA) for $\beta$-NGF has been established using its complemental DNA (hereinafter abbreviated as cDNA) as a probe [D. L. Shelton and L. F. Reichardt, Proc. Natl. Acad. Sci. USA, 81, 7951-7955 (1984); R. Heumann et al., EMBO J., 3, 3183-3189 (1984)].

By applying these procedures, a clear positive correlation has been demonstrated between the grade of sympathetic innervation in the peripheral nervous system and gene expression of NGF in the innervated tissue.

More surprisingly, NGF has also been detected in the central nervous system of rats, particularly in hippocampus, neocortex, and basal forebrain, e.g. septum, olfatory bulb, diagonal band of Broca, and nucleus basalis magnocellularis. In addition, its mRNA content has been found at a high level in the hippocampus and neocortex. On the other hand, the NGF content in the septum of the basal forebrain has been found at a low level as in other regions of the brain where no NGF antigen was detected [S. Korshing et al., EMBO J., 4, 1389-1393 (1985)]. Thereafter the results have been successively traced by other research groups [D. L. Shelton and L. F. Reichardt, Proc. Natl. Acad. Sci. USA, 83, 2714-2718 (1986); S. R. Whittemore et al., Proc. Natl. Acad. Sci. USA, 83, 817-821 (1986)].

According to these results, the NGF gene is expressed not only in the peripheral nervous system, but in the central nervous system as well. Furthermore, it was demonstrated that NGF is produced and secreted in the innervating regions of the chlorinergic tracts projecting from the origins of the basal forebrain to the neocortex and hippocampus, the centers of memory and learning, and then taken up at the nerve endings and transported in a retrograde manner through axons to reach somata in the origins. NGF has already been proven by a series of physiological experiments to be an essential factor for the survival and maintenance of functions in the chlorinergic tracts. These results have demonstrated the assumption that NGF has a specific function as a "neurotropic factor" also in the central nervous system. Thereafter the experiment has been traced by several research groups and has also been proven by investigation of NGF receptors and their distribution in the brain.

The present inventors have investigated the function of NGF as the neurotropic factor in the central nervous system. As discussed in the literature, summarized above, the disorders in memory and learning which are the early symptoms of SDAT are directly caused by the progressive degeneration of cholinergic tracts and consequent dysfunction of brain domains under their control.

The inventors, however, now believe that the failure of production and secretion of NGF in particular regions of brain can be the truly fundamental cause of early symptoms in SDAT. This is because conventional symptomatic trials against SDAT, such as supplementation and/or availability improvement therapies with acetylcholine, have been made without any remarkable result. On the other hand, it is believed that effective therapy may be realized if the functionally vicious cycle between responsible nerves and regions under their control could be broken by maintaining the production and secretion of NGF in the cerebral cortex and hippocampus.

Procedures for preparing human-type $\beta$-NGF in a large amounts have already been developed by gene-manipulation, yet many pharmacological and pharmaceutical limitations still exist on achieving supplemental therapy of NGF itself, which is a protein having a molecular weight of above 10,000. To date, there has been no application of NGF to the cetnral nervous system.

It is important from the above viewpoint to investigate low molecular weight compounds capable of inducing the production and secretion of NGF in particular tissues to be used as therapeutics for substantial and effective supplemental NGF therapy. The present inventors have already reported catechol derivatives having such activity (Ikeda: US Patent Application No. 07/098554). EPA 0,089,710 (Proctor and Gamble) discloses specific amide compounds as anti-irritants, but does not mention any induction of NGF secretion. There are also reports of Furukawa et al. [Y. Furukawa et al., J. Boil. Chem., 261, 6039 (1986) and FEBS Letters, 208, 258(1986)]

## Summary of the Invention

The object of this invention is to provide a medicine capable of inducing the production and secretion of NGF in particular tissues as a substantial and supplemental NGF therapy. That is, to provide a compound having, in regions under the control of specific nerves, acrivity for promoting the production and secretion of NGF which functions as a "neurotropic factor" for the responsible nerves to be administered by usual and convenient method. The compound is administered as it is or as a modified compound in accordance with customary pharmacological and pharmaceutical considerations. It is believed that the compound increases the supplied quantity of NGF into the locus of degenerated nerves and enables these nerves to recover their function. In particular, the compound is usuful for the treatment of SDAT, a disorder in of central nervous system for which fundamental therapy has not yet provided.

In the early onset stage of the SDAT symptoms, peripheral administration of the catechol derivative can enhance the NGF production and secretion ability in the cerebral cortex and hippocampus regions of the central nervous system. The progress of characteristic degeneration in the responsible cholinergic neuron is thereby inhibited. Repair of damaged neurons and reinnervation by surviving neurous are thus promoted.

Therefore this invention provides significant new therapy according to a new mechanism of action depending upon brain plasticity.

The present inventors have investigated low molecular weight compounds capable of inducing the production and secretion ability of NGF in specific tissue. As a result, it hs been found that a specific class of catechol derivatives have activity for inducing the production and secretion ability of NGF, and are effective to inhibit the progression and for therapy of regressive disorders of the central nervous system.

One aspect of this invention is a novel catechol derivative, or a pharmaceutically acceptable salt thereof, represented by the formula (I):

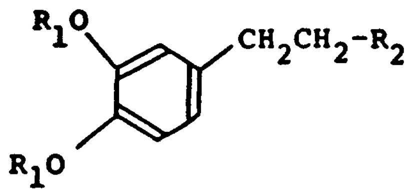

(I)

wherein $R_1$ is a hydrogen atom or an acetyl group and $R_2$ is a

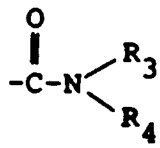

group or a

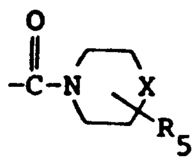

group, where $R_3$ and $R_4$ are each independently a hydrogen atom, an alkyl group, cycloalkyl group, aryl group or a benzyl group, and $R_5$ is a hydrogen atom, an alkyl group, aryl group, benzyl group, diphenylmethyl group or an alkoxycarbonyl group and X is a direct bond, oxygen atom, nitrogen atom or a methylene group provided that $R_3$ and $R_4$ are not both selected from the group consisting of H and $C_{1-3}$ alkyl groups.

Another aspect of this invention is prophylactic or therapeutic pharmaceutical preparation for the treatment of regressive disorders of the central nervous system which comprises as the active ingredient the catechol derivative according to the invention.

Detailed Description of the Preferred Embodiment

As examples of the substituent in the catechol derivative represented by the formula (I) of this invention, the alkyl group includes straight chain alkyl groups such as a methyl group, ethyl group, propyl group, butyl group, hexyl group, octyl group, decyl group, lauryl group, hexadecyl group, and a stearyl group, or branched chain alkyl groups such as an isopropyl group and an isobutyl group; the cycloalkyl group includes a cyclopropyl group, cyclobutyl group, cyclopentyl group, cyclohexyl group, and a cycloheptyl group; the aryl group includes a phenyl group and a naphthyl group; the alkoxycarbonyl group includes a methoxycarbonyl group and an ethoxycarbonyl group; and the alkyl group includes a methyl group, ethyl group and a propyl group.

Practical examples of the substituent having the formula (III):

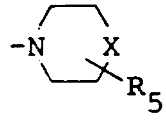

(III)

which is a part of the chemical structures represented by the formulas (I) and (II) are preferably a piperidino group, morpholino group, piperazino group, pyrrolidino group, 4-methylpiperazino group, 4-benzylpiperazino group, 4-diphenylmethanepiperazino group, prolyl group, nipecotinyl group and an isonipecotinyl group.

The method for preparing the compound of this invention will hereinafter be described.

The compounds of the invention can be readily prepared using conveniently available reactants such as dopamine, epinine and dihydrocaffic acid, and conducting chemical treatments such as the usual alkylation, acylation, esterification and conversion to amide (hereinafter as amidation). Novel compounds are prepared by the following methods.

In the first method, easily available dihydrocaffic acid ethyl ester is condensed by heating with the corresponding amine. The heating range is from room temperature to 200 °C, and in many cases, the reaction can proceed without a solvent. The reaction may be carried out, if necessary, by using the corresponding amine in excess or in inert solvents such as toluene and xylene.

In the second method, easily available dihydrocaffeic acid is converted to the diacetyl derivative by a normal method using acetic anhydride or acetyl chloride, and further reacted with thionyl chloride. The corresponding acid chloride thus obtained is then reacted with the corresponding amine in the presence of a base. The base which may be used is an organic base such as pyridine and triethylamine, an inorganic base such as sodium hydroxide and potassium hydroxide, or the corresponding amine which is present in excess. The preferred reaction temperature is in the range of 0-50 °C. The preferred solvents are the above organic base, water or organic solvents such as chloroform, tetrahydrofuran and benzene.

The effects of the various compounds of this invention as the preventive and therapeutic treatments for regressive disorders of the central nervous system were assessed by the following test. A mouse fibroblast cell line, L-M cells (ATCC, CCLI, 2) was used which was described in Y. Furukawa et al., J. Biol. Chem., 261, 6039 (1986). Concentration of NGF produced and secreted in the presence of the compound of this invention was measured by the highly sensitive ELISA method. The concentration of NGF was also measured in a system using astroglial cells which were considered as a major source for the production and secretion of NGF in the central nervous system. As shown below in detail in the examples, it has been found that the compound of this invention has an extremely high ability for promoting the production and secretion of NGF. As a result, it has been confirmed that the compounds of this invention may be useful as preventive and therapeutic preparations effective for regressive disorders in the central nervous system in general and SDAT in particular.

When a compound of this invention is used as the active ingredient in preventive or therapeutic pharmaceutical composition or preparation for regressive disorders of the central nervous system, dose and formulation are naturally different depending upon the physical properties of the particular compound, symptoms of the patient and other factors. In oral administration, a suggested dose for an adult is 50 - 1000 mg a day and may be given as a single dose or in divided doses in the form of tablets, granules, powders, suspensions or capsules. In non-oral administration, a dose of 1 - 100 mg may be given as a single dose or in divided doses in the form of injections, suppositories of isotonic solutions for infusion.

For example, in preparing tablets, crystalline cellulose, light anhydrous silicic acid and the like are used for adsorbents, and corn starch, lactose, calcium phosphate, magnesium stearate and the like are used for excipients. In preparing injections, the compound of this invention is used as an aqueous solution, an aqueous lyophobic solution in cotton seed oil, corn oil, peanut oil, olive oil etc. and also as an emulsion obtained by using surface active agents such as HCO-60 (hydrogenated caster oil, NIKKO CHEMICALS' trade name)

## Examples

The present invention will hereinafter be illustrated in detail with respect to the following examples; however, these examples are not to be construed as limiting the scope of the invention.

### Example 1

N-n-Pentyl-3,4-dihydroxyphenylpropionamide (Compound No. 86)

A mixture of 5 g of ethyl 3,4-diacetoxyphenylpropionate and 3.5 g of n-pentylamine was heated to 150 °C with stirring for 2 hours in an autoclave. After cooling, the reaction mixture was concentrated and the residue was purified by silica gel chromatography. A solvent mixture of chloroform:methanol = 20:1 was used as eluent. Pure N-n-pentyl-3,4-dihydroxyphenylpropionamie was obtained as an oily liquid.

NMR δ ppm ($CDCl_3$):

0.70-1.00 (m, 3H), 1.00-1.60 (m, 6H), 2.30-2.60 (m, 2H), 2.60-3.00 (m, 2H), 3.00-3.30 (m, 2H), 5.70 (br, 1H), 6.40-6.90 (m, 3H), 6.00-8.00 (br, 2H)

Example 2

N-n-Hexyl-3,4-diacetoxyphenylpropionamide (Compound No. 88)

To a solution of 2.0 g of N-n-hexyl-3,4-dihydroxyphenylpropionamide in 10 ml of pyridine, 3.4 g of acetic anhydride was added dropwise and heated to 65-70 °C with stirring for an hour. The reaction mixture was poured into 100 ml of ice water, neutralized with 25 ml of 6N hydrochloric acid and extracted three times with 50 ml of chloroform. The extracted solution was washed with a saturated aqueous sodium hydrogen carbonate solution and dried over anhydrous sodium sulfate. The solvent was distilled off under reduced pressure. Hexane was added to the residue. The separated crystals were filtered.
Melting point: 70-72 °C
NMR δ ppm ($CDCℓ_3$):
0.80-1.00 (m, 3H), 1.00-1.60 (m, 10H), 2.26 (s, 6H), 2.80-3.30 (m, 4H), 5.70 (br, 1H), 7.00-7.20 (m, 3H)

Example 3

N-n-Hexyl-3,4-dihydroxyphenylpropionamide (Compound No. 87)

Ethyl 3,4-diacetoxyphenylpropionate and n-hexylamine were used to obtain N-n-Hexyl-3,4-dihydroxyphenylpropionamide by carrying out the same procedures as described in Example 1.
Melting point: 69-70 °C
NMR δ ppm (DMSO-$d_6$):
0.80-1.00 (m, 3H), 1.26 (s, 10H), 2.16-2.48 (m, 2H), 2.80-3.20 (m, 2H), 6.36-6.70 (m, 3H), 7.64-7.84 (m, 1H), 8.60 (br, 2H)

Example 4

N-Lauryl-3,4-dihydroxyphenylpropionamide (Compound No. 89)

Ethyl 3,4-diacetoxyphenylpropionate and laurylamine were used to obtain N-Lauryl-3,4-dihydroxyphenylpropionamide by carrying out the same procedures as described in Example 1.
Melting point: 100-102 °C
NMR δ ppm (DMSO-$d_6$):
0.80-1.00 (m, 3H), 1.24 (s, 22H), 2.20-2.48 (m, 2H), 2.90-3.20 (m, 2H), 6.40-6.76 (m, 3H), 7.72 (br, 1H), 8.40-8.80 (m, 2H)

Example 5

N-n-Butyl-N-methyl-3,4-dihydroxyphenylpropionamide (Compound No. 90)

Ethyl 3,4-diacetoxyphenylpropionate and N-methyl-n-butylamine were used to obtain N-n-Butyl-N-methyl-3,4-dihydroxyphenylpropionamide by carrying out the same procedures as described in Example 1.
Melting point: 96-98 °C
NMR δ ppm ($CDCℓ_3$):
0.38-0.80 (m, 38), 0.80-1.40 (m, 4H), 2.03-2.53 (m, 4H), 2.53-2.80 (m, 3H), 2.80-3.33 (m, 2H), 6.06-6.70 (m, 3H), 6.70-7.53 (br, 2H)

Example 6

N-Stearyl-3,4-dihydroxyphenylpropionamide (Compound No. 91)

Ethyl 3,4-diacetoxyphenylpropionate and stearylamine were used to obtain N-Stearyl-3,4-dihydroxyphenylpropionamide by carrying out the same procedures as described in Example 1.
Melting point: 100-102 °C
NMR δ ppm (DMSO-$d_6$):
0.80-1.00 (m, 3H), 1.24 (s, 30H), 2.10-2.40 (m, 4H), 2.40-2.60 (m, 2H), 2.80-3.10 (m, 2H), 6.30-6.68 (m, 3H), 7.70 (br, 1H), 8.50 (s, 1H), 8.70 (s, 1H)

Example 7

N,N-Di-n-hexyl-3,4-dihydroxyphenylpropionamide (Compound No. 92)

Ethyl 3,4-diacetoxyphenylpropionate and di-n-hexylamine were used to obtain N,N-Di-n-hexyl-3,4-dihydroxyphenylpropionamide by carrying out the same procedures as described in Example 1, as an oily product.
NMR δ ppm ($CDCl_3$):
0.60-1.00 (m, 6H), 1.00-1.60 (m, 16H), 2.40-3.00 (m, 4H), 3.00-3.40 (m, 4H), 6.10-7.00 (m, 5H)

Example 8

N-Cyclohexyl-3,4-dihydroxyphenylpropionamide (Compound No. 93)

Ethyl 3,4-diacetoxyphenylpropionate and cyclohexylamine were used to obtain N-Cyclohexyl-3,4-dihydroxyphenylpropionamide by carrying out the same procedures as described in Example 1 as an fatty product.
NMR δ ppm ($CDCl_3$):
0.10-2.00 (m, 11H), 2.20-2.60 (m, 2H), 2.60-2.90 (m, 2H), 5.40-5.60 (m, 1H), 6.00-7.00 (m, 3H), 8.10 (br, 1H)

Example 9

N-Methyl-N-benzyl-3,4-dihydroxyphenylpropionamide (Compound No. 94)

Ethyl 3,4-diacetoxyphenylpropionate and N-methylbenzylamine were used to obtain N-Methyl-N-benzyl-3,4-dihydroxyphenylpropionamide by carrying out the same procedures as described in Example 1.
Melting point: 107-109 °C
NMR δ ppm ($CDCl_3$):
2.40-3.00 (m, 7H), 4.50 (s, 2H), 6.40-7.80 (m, 10H)

Example 10

N-Phenyl-3,4-dihydroxyphenylpropionamide (Compound No. 95)

Ethyl 3,4-diacetoxyphenylpropionate and aniline were used to obtain N-Phenyl-3,4-dihydroxyphenylpropionamide by carrying out the same procedures as described in Example 1 as an oily product.
NMR δ ppm (DMSO-$d_6$):
2.40-2.90 (m, 4H), 6.30-6.80 (m, 3H), 6.80-7.70 (m, 5H), 8.50 (br, 2H), 9.76 (s, 1H)

Example 11

N-Adamantyl-3,4-dihydroxyphenylpropionamide (Compound No. 96)

Ethyl 3,4-diacetoxyphenylpropionate and 1-aminoadamantane were used to obtain N-Adamantyl-3,4-dihydroxyphenylpropionamide by carrying out the same procedures as described in Example 1.
Melting point: 213-215 °C
NMR δ ppm (DMSO-$d_6$):
1.60 (m, 6H), 1.80-2.10 (m, 8H), 2.10-2.30 (m, 2H), 2.40-2.70 (m, 3H), 6.30-6.60 (m, 3H), 8.40-8.80 (br, 2H)

Example 12

N-[3-(3,4-Dihydroxyphenyl)propionyl]piperidine (Compound No. 97)

A mixture of 4.2 g of ethyl 3,4-diacetoxyphenylpropionate and 2.6 g of piperidine was heated to 150 °C with stirring for 4 hours in an autoclave. After cooling, the reaction mixture was concentrated. The residue was purified by column chromatography using silica gel. A solvent mixture of chloroform:methanol = 10:1 was used as eluent. The corresponding fraction was concentrated. The resultant residue was crystallized from a solvent mixture of hexane and ether. The separated crystals were filtered.

Pure N-[3-(3,4-Dihydroxyphenyl)propionyl]piperidine was obtained as colorless crystals. The yield was calculated as 4.3 g.
Melting point: 114-115 °C
NMR δ ppm (DMSO-$d_6$):
1.2-1.6 (br, 6H), 2.3-2.7 (m, 4H), 3.2-3.5 (m, 4H), 6.25-6.55 (m, 3H), 8.5 (br, 2H)

Example 13

N-[3-(3,4-Dihydroxyphenyl)propionyl]pyrrolidine (Compound No. 98)

Ethyl 3,4-diacetoxyphenylpropionate and pyrrolidine were used to obtain N-[3-(3,4-Dihydroxyphenyl)-propionyl]pyrrolidine by carrying out the same procedures as described in Example 12.
Melting point: 172-173 °C
NMR δ ppm (DMSO-$d_6$):
1.50-2.00 (m, 4H), 2.20-2.80 (m, 4H), 3.10-3.40 (m, 4H), 6.30-6.70 (m, 3H), 8.50 (br, 1H)

Example 14

N-[3-(3,4-Dihydroxyphenyl)propionyl]morpholine (Compound No. 99)

Ethyl 3,4-diacetoxyphenylpropionate and morpholine were used to obtain N-[3-(3,4-Dihydroxyphenyl)-propionyl]morpholine by carrying out the same procedures as described in Example 12.
Melting point: 211-213 °C
NMR δ ppm (DMSO-$d_6$):
2.4-2.7 (m, 4H), 3.2-3.6 (m, 8H), 6.25-6.60 (m, 3H), 8.40 (s, 1H), 8.48 (s, 1H)

Example 15

N-Methyl-N'-[3-(3,4-dihydroxyphenyl)propionyl]piperazine (Compound No. 100)

3,4-Dihydroxyphenylpropionic acid ethyl ester and N-methylpiperazine were used to obtain N-Methyl-N'-[3-(3,4-dihydroxyphenyl)propionyl]piperazine by carrying out the same procedures as described in Example 12.
Melting point: 190-193 °C
NMR δ ppm (DMSO-$d_6$):
2.00-2.40 (m, 7H), 2.40-2.80 (m, 3H), 3.20-3.60 (m, 5H), 6.40-6.70 (m, 3H), 8.60 (br, 2H)

Example 16

N-Benzyl-N'-[3-(3,4-dihydroxyphenyl)propionyl]piperazine (Compound No. 101)

Ethyl 3,4-diacetoxyphenylpropionate and N-benzylpiperazine were used to obtain N-Benzyl-N'-[3-(3,4-dihydroxyphenyl)propionyl]piperazine by carrying out the same procedures as described in Example 12.
Melting point: 178-180 °C
NMR δ ppm (DMSO-$d_6$):
2.20-2.40 (m, 4H), 2.40-2.70 (m, 4H), 3.20-3.60 (m, 6H), 6.30-6.80 (m, 3H), 7.20-7.56 (m, 4H), 8.56 (s, 2H)

Example 17

N-Diphenylmethyl-N'-[3-(3,4-dihydroxyphenyl)propionyl]piperazine (Compound No. 102)

Ethyl 3,4-diacetoxyphenylpropionate and N-diphenylmethylpiperazine were used to obtain N-Diphenylmethyl-N'-[3-(3,4-dihydroxyphenyl)propionyl]piperazine by carrying out the same procedures as described in Example 12.
Melting point: 163-164 °C
NMR δ ppm ($CDC\ell_3$):
2.00-3.00 (m, 8H), 3.10-3.70 (m, 4H), 4.12 (s, 1H), 6.00-7.60 (m, 13H)

Example 18

N-[3-(3,4-dihydroxyphenyl)propionyl]-L-proline methyl ester (Compound No. 103)

(1) To a solution of 2 g of 3,4-diacetylphenylpropionic acid in 10 ml of chloroform, 8.9 g of thionyl chloride was added and heated to 50 ° C with stirring for 2 hours. The solvent was distilled off under reduced pressure. The residue thus obtained was crude 3,4-diacetylphenylpropionyl chloride. The yield was 2.1 g. The crude propionyl chloride was used for the next reaction without further purification.

(2) To a suspension of 1.49 g of L-proline methyl ester in 15 ml of chloroform, 3 g of triethylamine was added dropwise. The propionyl chloride obtained in (1) was dissolved in 7 ml of chloroform and added dropwise to the above mixture under ice cooling. The resultant mixture was allowed to stand overnight and heated to 50 ° C with stirring for an hour. After cooling, the reaction mixture was poured into 10 ml of ice water. The chloroform layer was separated, washed twice with 25 ml of one N hydrochloric acid and further washed once with an aqueous sodium chloride solution. The resultant chloforform solution was dried over anhydrous sodium sulfate and concentrated. The residue was purified by column chromatography using silica gel. A solvent mixture of chloroform:methanol = 50:1 was used as eluent. N-[3-(3,4-Dihydroxyphenyl)propionyl]-L-proline methyl ester was obtained as colorless oily product. The yield was 1.25 g.

NMR $\delta$ ppm ($CDC\ell_3$):

1.60-2.40 (m, 4H), 2.12 (s, 3H), 2.30 (s, 3H), 2.40-3.10 (m, 4H), 3.72 (s, 3H), 3.20-3.90 (m, 2H), 4.32-4.50 (m, 1H), 6.60-7.20 (m, 3H)

(3) To a solution of 1.0 g of N-[3-(3,4-diacetoxyphenyl)propionyl]-L-proline methyl ester in 5 ml of methanol, 10 ml of a 5% aqueous ammonia solution was added under ice cooling, and stirred for an hour. The reaction mixture was then acidified with 6N hydrochloric acid under ice cooling and extracted twice with chloroform. The chloroform solution was dried over anhydrous sodium sulfate. N-[3-(3,4-diacetoxyphenyl)propionyl]-L-proline methyl ester was obtained after distilling off chloroform as a colorless oily product. The yield was 0.7 g.

NMR $\delta$ ppm ($CDC\ell_3$):

1.60-2.30 (m, 4H), 2.30-3.00 (m, 4H), 3.00-3.60 (m, 2H), 3.64 (s, 3H), 4.40-4.60 (m, 1H), 6.40-6.80 (m, 3H), 6.90 (br, 1H), 7.60 (br, 1H)

Example 19

Ethyl N-[3-(3,4-dihydroxyphenyl)propionyl]nipecotinate (Compound No. 104)

The same procedures as described in Example 18 were carried out except that nipecotic acid ethyl ester hydrochloride was used in place of L-proline methyl ester hydrochloride.

Thus N-[3-(3,4-dihydroxyphenyl)propionyl]nipecotic acid ethyl ester was obtained as a colorless oily product.

NMR $\delta$ ppm ($CDC\ell_3$):

1.20-1.48 (m, 3H), 1.46-2.40 (m, 5H), 2.40-3.54 (m, 6H), 3.54-4.68 (m, 4H), 6.50-7.20 (m, 4H), 7.66 (br, 1H)

Example 20

Acute toxicity

A group of male ddy 10 mice 5-weeks old was used for the acute toxicity test. The test sample was prepared by suspending the test compound in 0.5% aqueous Tween 80. The test compound was administered by intraperitoneal injection. Numbers of deaths were counted after one and four days, and an $LD_{50}$ value was calculated from these numbers.

None of the compounds tested had a $LD_{50}$ value of at least 1000 mg/kg. That is, acute toxicity was extremely low.

Example 21

Promoting activity for the production and secretion of NGF in mouse L-M cells

This experiment was performed according to the procedures of Y. Furukawa et al. which is described in J. Biol. Chem., 261, 6039 - 6047 (1986).

Mouse L-M cells were precultured in Medium 199 (a product of Gibco Co.) supplemented with 0.5% peptone, and then innoculated in a 24-well cultivation plate having a well surface area of 2.1 $cm^2$ (a product of Falcon Co.) at a cell density of about 3 x $10^4$ cells/well. The medium was cultured for 3 days at a temperature of 37 °C. After completing the confluency (about $10^6$ cells/well), the medium was changed to Medium 199 (0.5 ml/well) containing 0.5% bovine serum albumin (Fraction V, a product of Armour Co.). The sample of the derivative tested is contained in the medium at a prescribed concentration as illustrated in the Tables. NGF concentration in the medium after cultivating for 24 hours was determined according to high sensitivity ELISA [S. Furukawa et al., J. Nuurochem., 40, 734 - 744 (1983)]. Data are expressed as fold increase in NGF content of the medium over that cultivated in the absence of the derivative to be tested. The lower detection limit of ELISA is 0.25 pg/ml and the NGF content of control medium is normally 50-200 pg/0.5 ml/well. Data are presented as the mean of four determinations. The results are illustrated in Table 5.

Example 22

Promoting activity for the production and secretion of NGF in mouse brain astroglial cells

The experiment was performed by inducing astroglial cells from the mouse forebrain to a culture system according to the procedures of S. Furukawa et al. which is described in Biochem. Biophys. Res. Commun., 136, 57 - 63 (1986).

Forebrains of 8-days old mice were dissected out and cut into small pieces. The pieces were washed with calcium- and magnesium-free phosphate-buffered saline (hereinafter abbreviated as PBS), treated with 0.25% trypsin containing PBS at 37 °C for 30 minutes and triturated with a Pasteur pipet to give a suspension. Cells and cell clumps were recovered by centrifugation at 200 xg for 5 minutes. They were cultured in Dulbecco modified Eagle's medium (a product of Gibco Co. hereinafter abbreviated as DMEM) containing 10% fetal calf serum, 50 µ units/ml of penicillin, and 50 µg/ml of streptomycin, for 10 to 14 days with medium changes every 3 days. After completing confluency, the cells were dissociated by trypsin treatment and recultured in new culture flasks. This procedure was repeated further twice and more. The culture became a uniform cell cluster. The cell cluster for use in this invention can be stained not less than 97% in accordance with the PAP staining method (peroxidase/antioxidase staining method) using anti-human glial fibrillar acidic protein (GFAP) rabbit antiserum. The cells will hereinafter be referred to as astroglial cells.

Astroglial cells were innoculated in 24-well plates having a well surface area of 2.1 $cm^2$ (a product of Falcon Co.) at a cell density of about 3 x $10^4$ cells/well and cultured for 3 days in DMEM medium supplemented with 10% of fetal calf serum. After completing confluency about($10^7$ cells/well), the medium was changed to DMEM medium (0.5 ml/well) supplemented with 0.5% of bovine serum albumin (fraction V) and cultured for 3 days. The culture was further continued with medium changes every 3 days. After cells were practically synchronized in the quiscent stage, the medium was changed to 0.5 ml of the same medium and containing a prescribed concentration of test sample as illustrated in the Tables. NGF in the medium after cultivating for 24 hours was determined by the ELISA as mentioned above. Data are expressed as fold increase in NGF content over that in the absence of the test sample. The lower detection limit of the ELISA is 0.25 pg/ml and the NGF content of control medium was normally 1-10 pg/0.5 ml/well. Data are presented as the mean of four determinations. The results are illustrated in Table 5.

### Table 5

Promoting activity of compound having the following formula for the production and secretion of NGF in mouse L-M cells and brain astroglial cells.

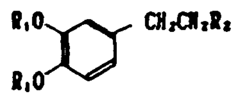

| Compound | | | | NGF | | | |
|---|---|---|---|---|---|---|---|
| No. | Substituent | | Concentration | L-M Cell | | Astroglial Cell | |
| | $R_1$ | $R_2$ | (mM) | Concentration (ng/well) | Incrase Ratio | Concentration (ng/well) | Incrase Ratio |
| 86 | H | $-CONH(CH_2)_4CH_3$ | 0.4 | 5.66 | 6.5 | 493.9 | 30.3 |
| 87 | H | $-CONH(CH_2)_5CH_3$ | 0.4 | 5.05 | 5.8 | 195.6 | 12.0 |
| 88 | $CH_3CO$ | $-CONH(CH_2)_5CH_3$ | 0.4 | 5.39 | 6.2 | 257.5 | 15.8 |
| 89 | H | $-CONH(CH_2)_{11}CH_3$ | 0.4 | 3.74 | 4.3 | 81.5 | 5.0 |
| 90 | H | 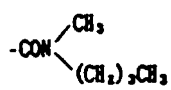 | 0.4 | 5.57 | 6.4 | 489.0 | 30.0 |
| 91 | H | $-CONH(CH_2)_{17}CH_3$ | 0.4 | 3.57 | 4.1 | 84.8 | 5.2 |
| 92 | H | 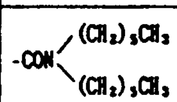 | 0.4 | 4.35 | 5.0 | 143.4 | 8.8 |
| 93 | H | 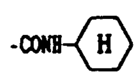 | 0.4 | 5.22 | 6.0 | 161.4 | 9.9 |
| 94 | H | 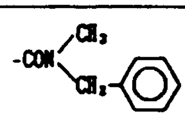 | 0.4 | 4.44 | 5.1 | 84.8 | 5.2 |
| 95 | H | 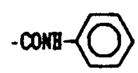 | 0.4 | 5.13 | 5.9 | 182.6 | 11.2 |
| 96 | H | 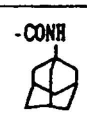 | 0.4 | 4.96 | 5.7 | 293.4 | 18.0 |
| 97 | H | 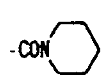 | 0.4 | 5.39 | 6.2 | 435.7 | 13.7 |
| 98 | H | 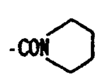 | 0.4 | 5.31 | 6.1 | 407.0 | 12.8 |

Table 5 (continued)

| Compound | | | | N G F | | | |
|---|---|---|---|---|---|---|---|
| No. | Substiuent | | Concentration | L-M Cell | | Astroglial Cell | |
| | $R_1$ | $R_2$ | (mM) | Concentration (ng/well) | Incrase Ratio | Concentration (ng/well) | Incrase Ratio |
| 99 | H | 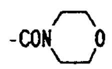 | 0.4 | 4.18 | 4.8 | 165.4 | 5.2 |
| 100 | H | 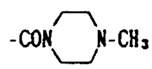 | 0.4 | 4.35 | 5.0 | 260.8 | 8.2 |
| 101 | H | 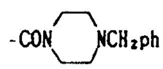 | 0.4 | 4.52 | 5.2 | 305.3 | 9.6 |
| 102 | H | 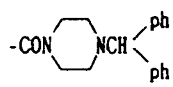 | 0.4 | 4.44 | 5.1 | 321.2 | 10.1 |
| 103 | H | 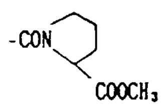 | 0.4 | 3.74 | 4.3 | 200.3 | 6.3 |
| 104 | H | 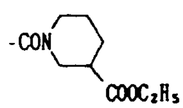 | 0.4 | 4.96 | 5.7 | 248.0 | 7.8 |
| Control | | | 0 | 0.87 | 1.00 | 31.8 | 1.00 |

注) (1) ph : phenyl group

## Claims

**1.** A catechol derivative or a pharmaceutically acceptable salt thereof represented by the formula (I):

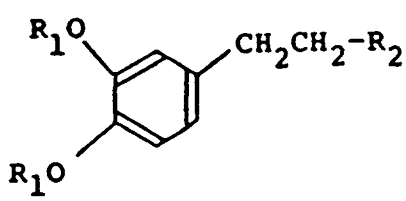

(I)

wherein $R_1$ is a hydrogen atom or an acetyl group and $R_2$ is a

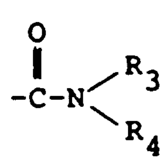

group or a

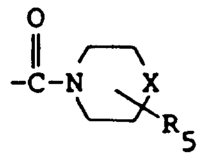

group, where $R_3$ and $R_4$ are each independently a hydrogen atom, an alkyl group, cycloalkyl group, aryl group or a benzyl group, and $R_5$ is a hydrogen atom, an alkyl group, aryl group, benzyl group, diphenylmethyl group or an alkoxycarbonyl group and X is a direct bond, oxygen atom, nitrogen atom or a methylene group provided that $R_3$ and $R_4$ are not both selected from the group consisting of H and $C_{1-3}$ alkyl groups.

**2.** A pharmaceutical composition for the treatment of regressive disorders of the central nervous system which comprises as an active ingredient a catechol derivative according to claim 1.

**3.** The catechol derivative of claim 1 in which the derivative is selected from the group consisting of those numbered below 86 to 104;

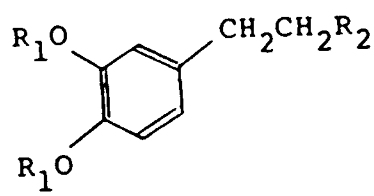

| No. | Substiuent | |
|---|---|---|
| | $R_1$ | $R_2$ |
| | | |
| 86 | H | $-CONH(CH_2)_4CH_3$ |
| 87 | H | $-CONH(CH_2)_5CH_3$ |
| 88 | $CH_3CO$ | $-CONH(CH_2)_5CH_3$ |
| 89 | H | $-CONH(CH_2)_{11}CH_3$ |
| 90 | H | 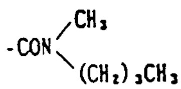 |
| 91 | H | $-CONH(CH_2)_{17}CH_3$ |
| 92 | H | 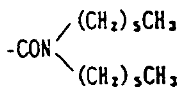 |
| 93 | H | 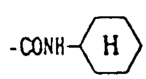 |
| 94 | H | 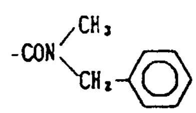 |
| 95 | H | 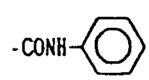 |
| 96 | H | 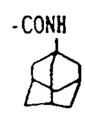 |

| No. | Substiuent | |
|---|---|---|
| | $R_1$ | $R_2$ |
| 97 | H | 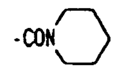 |
| 98 | H | 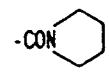 |
| 99 | H | 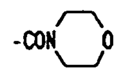 |
| 100 | H | 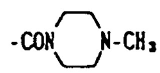 |
| 101 | H | 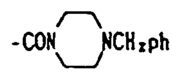 |
| 102 | H | 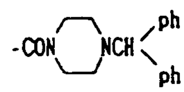 |
| 103 | H | 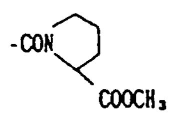 |
| 104 | H | 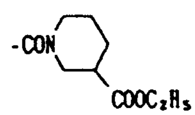 |

ph : phenyl group

**4.** The pharmaceutical composition of claim 2 containing from 50 to 1,000 mg of the catechol derivative.

**5.** The pharmaceutical composition of claim 2 or claim 4 in an orally administrable form.

**6.** The pharmaceutical composition of claim 2 in an injectable form containing from 1 to 100 mg of the catechol derivative.

**7.** A catechol derivative of claim 1 or claim 3; or a pharmaceutical composition of any one of claims 2, 4, 5 and 6; for use in therapy.

**8.** Use of a catechol derivative of claim 1 or claim 3; or a composition of any one of claims 2, 4, 5 and 6; for the manufacture of a medicament for use in treating regressive disorders of the central nervous system, by administering for example from 1 to 1,000 mg per day of the catechol derivative.

**9.** A catechol derivative of claim 7 for use in treating regressive disorders of the central nervous system, by administering for example from 1 to 1,000 mg per day of the catechol derivative.

**10.** Use according to claim 8 or a catechol derivative of claim 9 wherein the regressive disorder of the central nervous system is senile dementia of the Alzheimer type.

**Patentansprüche**

**1.** Derivat des Catechins oder eines seiner pharmazeutisch verträglichen Salze, dargestellt durch die Formel (I):

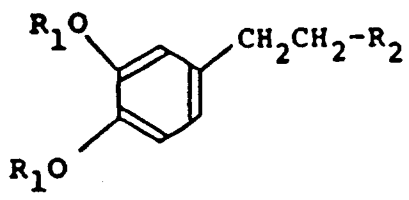

(I)

wobei $R_1$ ein Wasserstoffatom oder eine Acetyl-Gruppe und $R_2$ eine

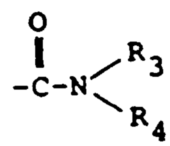

-Gruppe oder eine

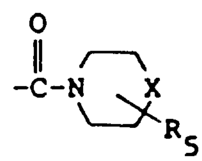

-Gruppe ist, wobei $R_3$ und $R_4$ jeweils unabhängig voneinander ein Wasserstoff-Atom, eine Alkyl-Gruppe, eine Cycloalkyl-Gruppe, eine Aryl-Gruppe oder eine Benzyl-Gruppe sind, wobei $R_5$ ein Wasserstoff-Atom, eine Alkyl-Gruppe, eine Aryl-Gruppe, eine Benzyl-Gruppe, eine Diphenylmethyl-Gruppe oder eine Alkoxycarbonyl-Gruppe ist, und wobei X eine direkte Bindung, ein Sauerstoff-Atom, ein Stickstoff-Atom oder eine Methylen-Gruppe ist, wobei die Voraussetzung gilt, daß $R_3$ und $R_4$ nicht beide ausgewählt sind aus der Gruppe, die aus H und $C_{1-3}$-Alkyl-Gruppen besteht.

**2.** Pharmazeutische Zusammensetzung für die Behandlung von regressiven Störungen des zentralen Nervensystems, das als Wirkstoff ein Derivat des Catechins nach Anspruch 1 enthält.

**3.** Das Derivat des Catechins nach Anspruch 1, wobei das Derivat ausgewählt ist aus der Gruppe, die aus den Verbindungen besteht, die im nachfolgenden mit den Nummern 86 bis 104 aufgelistet sind:

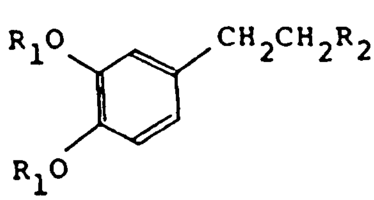

| No. | Substiuent | |
|---|---|---|
| | $R_1$ | $R_2$ |
| | | |
| 86 | H | $-CONH(CH_2)_4CH_3$ |
| 87 | H | $-CONH(CH_2)_5CH_3$ |
| 88 | $CH_3CO$ | $-CONH(CH_2)_5CH_3$ |
| 89 | H | $-CONH(CH_2)_{11}CH_3$ |
| 90 | H | 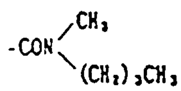 |
| 91 | H | $-CONH(CH_2)_{17}CH_3$ |
| 92 | H | 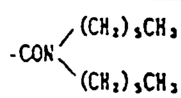 |
| 93 | H | 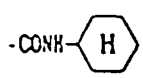 |
| 94 | H | 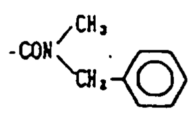 |
| 95 | H | 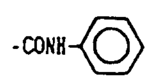 |
| 96 | H | 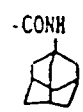 |

| No. | Substiuent | |
|---|---|---|
| | $R_1$ | $R_2$ |
| 97 | H | 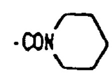 |
| 98 | H | 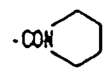 |
| 99 | H | 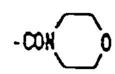 |
| 100 | H | 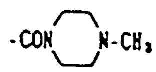 |
| 101 | H | 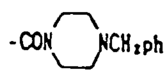 |
| 102 | H | 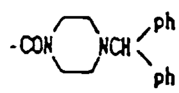 |
| 103 | H | 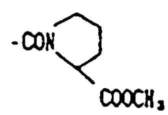 |
| 104 | H | 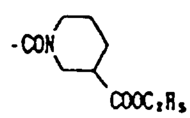 |

ph: Phenyl-Gruppe

**4.** Die pharmazeutische Zusammensetzung nach Anspruch 2, die 50 bis 1000 mg des Derivats des Catechins enthält.

**5.** Die pharmazeutische Zusammensetzung nach Anspruch 2 oder 4 in einer oral verabreichbaren Form.

**6.** Die pharmazeutische Zusammensetzung nach Anspruch 2 in einer injizierbaren Form, die 1 bis 100 mg des Derivats des Catechins enthält.

**7.** Das Derivat des Catechins nach Anspruch 1 oder 3, die pharmazeutische Zusammensetzung nach einem der Ansprüche 2, 4, 5 oder 6 für die Verwendung in der Therapie.

**8.** Verwendung des Derivats des Catechins nach Anspruch 1 oder 3; oder eine Zusammensetzung nach einem der Ansprüche 2, 4, 5 oder 6 für die Herstellung eines Medikaments zur Verwendung für die Behandlung von regressiven Störungen des zentralen Nervensystems durch Verabreichung von zum Beispiel 1 bis 1000 mg pro Tag des Derivats des Catechins.

**9.** Das Catechin-Derivat nach Anspruch 7 für die Verwendung zur Behandlung von regressiven Störungen des zentralen Nervensystems durch Verabreichung von zum Beispiel von 1 bis 1000 mg pro Tag des Derivats des Catechins.

**10.** Die Verwendung nach Anspruch 8 oder das Derivat des Catechins nach Anspruch 9, wobei die regressive Störung des zentralen Nervensystems die senile Demenz des Alzheimer-Typs ist.

**Revendications**

**1.** Dérivé du catéchol ou un de ses sels acceptables sur le plan pharmaceutique, représenté par la formule (I):

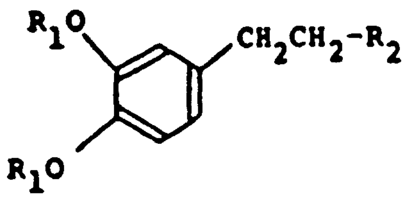

(I)

dans laquelle $R_1$ est un atome d'hydrogène ou un groupe acétyle et $R_2$ est un groupe

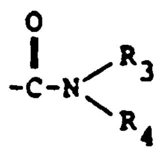

ou un groupe

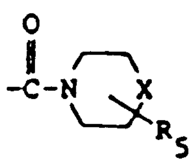

où $R_3$ et $R_4$ représentent chacun indépendamment un atome d'hydrogène, un groupe alkyle, un groupe cycloalkyle, un groupe aryle ou un groupe benzyle, et $R_5$ est un atome d'hydrogène, un groupe alkyle, un groupe aryle, un groupe benzyle, un groupe diphénylméthyle ou un groupe alkoxycarbonyle et X est une liaison directe, un atome d'oxygène, un atome d'azote ou un groupe méthylène à condition que $R_3$ et $R_4$ ne soient pas tous deux choisis dans le groupe formé par un atome H et les groupes alkyle en $C_1$ à $C_3$.

**2.** Composition pharmaceutique pour le traitement des troubles régressifs du système nerveux central qui comprend comme principe actif un dérivé du catéchol selon la revendication 1.

**3.** Dérivé du catéchol selon la revendication 1, dans lequel le dérivé est choisi dans le groupe formé par ceux numérotés ci-dessous de 86 à 104:

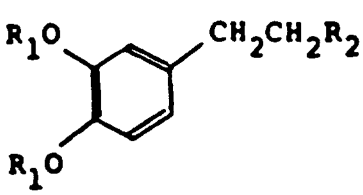

| No. | Substituant | |
|---|---|---|
| | $R_1$ | $R_2$ |
| | | |
| 86 | H | $-CONH(CH_2)_6CH_3$ |
| 87 | H | $-CONH(CH_2)_3CH_3$ |
| 88 | $CH_3CO$ | $-CONH(CH_2)_3CH_3$ |
| 89 | H | $-CONH(CH_2)_{11}CH_3$ |
| 90 | H | 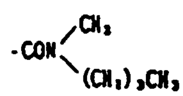 |
| 91 | H | $-CONH(CH_2)_{17}CH_3$ |
| 92 | H | 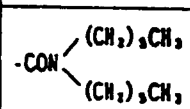 |
| 93 | H | 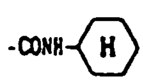 |
| 94 | H | 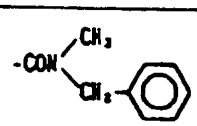 |
| 95 | H | 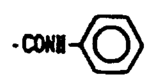 |
| 96 | H | 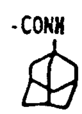 |

| No. | Substituant | |
|---|---|---|
| | $R_1$ | $R_2$ |
| 97 | H | 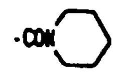 |
| 98 | H | 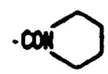 |
| 99 | H | 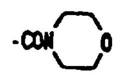 |
| 100 | H | 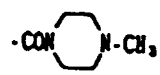 |
| 101 | H | 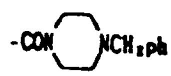 |
| 102 | H | 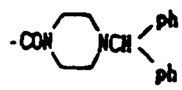 |
| 103 | H | 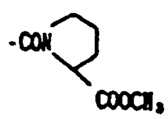 |
| 104 | H | 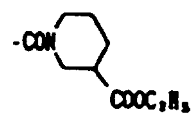 |

ph: groupe phényle

4. Composition pharmaceutique selon la revendication 2, contenant de 50 à 1 000 mg du dérivé du catéchol.

5. Composition pharmaceutique selon la revendication 2 ou 4 sous une forme administrable par voie orale.

6. Composition pharmaceutique selon la revendication 2 sous une forme injectable contenant de 1 à 100 mg du dérivé du catéchol.

7. Dérivé du catéchol selon la revendication 1 ou 3; ou composition pharmaceutique selon l'une quelconque des revendications 2, 4, 5 et 6; pour une utilisation en thérapie.

8. Utilisation d'un dérivé du catéchol selon la revendication 1 ou 3; ou composition pharmaceutique selon l'une quelconque des revendications 2, 4, 5 et 6; pour la fabrication d'un médicament en vue d'une utilisation dans le traitement des troubles régressifs du système nerveux central, en administrant par exemple de 1 à 1 000 mg par jour du dérivé du catéchol.

9. Dérivé du catéchol selon la revendication 7 en vue d'une utilisation dans le traitement des troubles régressifs du système nerveux central, en administrant par exemple de 1 à 1 000 mg par jour du dérivé du catéchol.

10. Utilisation selon la revendication 8 ou dérivé du catéchol selon la revendication 9, dans lequel le trouble régressif du système nerveux central est la démence sénile de type Alzheimer.